# DEMANDE DE BREVET EUROPEEN

(11) **EP 1 038 518 A1**
(43) Date de publication de la demande: **27.09.2000**
(21) Numéro de dépôt: 00400531.0
(22) Date de dépôt: 28.02.2000
(51) Int. Cl.: A61K 7/48

(54) **Composition amincissante comprenant un polymère filmogène hydrosoluble**

(30) Priorité: 22.03.1999 FR 9903532
(71) Demandeur: L'OREAL, 75008 Paris (FR)
(72) Inventeur: Picard, Elisabeth, 78140 Velizy (FR)
(74) Mandataire: Rasson, Catherine

(57) **Abrégé**

L'invention concerne une composition amincissante sous forme de spray, comprenant au moins un monoalcool en C₁-C₆, au moins un polymère filmogène hydrosoluble et au moins un actif amincissant.

Le polymère filmogène peut être notamment choisi parmi les polymères anioniques, amphotères, non ioniques et leurs mélanges.

Ladite composition est particulièrement appropriée pour lutter contre l'adiposité, la cellulite et/ou la peau d'orange, et/ou obtenir un effet d'amincissement du corps et/ou du visage.

## Description

La présente invention se rapporte à une composition amincissante sous forme de spray, contenant au moins un polymère filmogène hydrosoluble et au moins un actif amincissant, et à son utilisation notamment pour lutter contre l'adiposité, la cellulite et/ou la peau d'orange, et/ou obtenir un effet d'amincissement du corps et/ou du visage.

L'adiposité (ou excès de graisse dans le tissu cellulaire sous-cutané) peut avoir de nombreuses causes plus ou moins complexes, plus ou moins connues ou plus ou moins comprises.

Certaines cellules de la peau, appelées adipocytes, contiennent des quantités variables de graisses sous la forme de triglycérides, ces triglycérides étant synthétisés *in vivo* par les adipocytes eux-mêmes, selon des réactions de type enzymatique (lipogénèse), à partir des acides gras libres et du glucose (après dégradation de ce dernier en glycérol) contenus dans l'organisme et apportés à celui-ci par l'intermédiaire de certains aliments. Or, parallèlement, les triglycérides ainsi formés, puis stockés, dans les cellules adipocytaires peuvent également se redécomposer, toujours sous l'action d'enzymes spécifiques (lipolyse) contenues dans ces mêmes cellules, en libérant cette fois des acides gras d'une part et du glycérol et/ou des mono- et/ou des di-esters du glycérol d'autre part. Les acides gras ainsi relargués peuvent alors soit diffuser dans l'organisme pour y être consommés ou transformés de différentes façons, soit être recaptés (aussitôt ou un peu plus tard) par les adipocytes pour générer à nouveau des triglycérides par lipogénèse.

Si, pour des raisons diverses (nourriture trop riche, inactivité, vieillissement et autres), un déséquilibre substantiel s'installe dans l'organisme entre la lipogénèse (formation de triglycérides par réaction enzymatique entre des acides gras et le glycérol provenant du glucose) et la lipolyse (décomposition enzymatique de triglycérides en acides gras et glycérol), c'est à dire plus précisément si les quantités de graisses formées par lipogénèse deviennent notablement et constamment supérieures à celles qui sont éliminées par lipolyse, il se produit alors dans les adipocytes une accumulation de triglycérides qui, si elle devient excessive, peut se traduire progressivement par l'apparition d'une peau épaisse, à surface souvent irrégulière ("peau d'orange") et de consistance plus ou moins flasque ou gélatineuse, donnant finalement à la silhouette un aspect général disgracieux pouvant évoluer entre la simple surcharge locale (lipodismorphie) et la formation de cellulite, en passant par l'embonpoint certain, et enfin la réelle obésité.

Or, compte tenu notamment du profond inconfort tant physique qu'esthétique, et parfois psychologique, qu'elles occasionnent auprès des individus qui en sont atteints, en particulier chez les femmes, l'adiposité et la cellulite constituent de nos jours une affection de moins en moins bien supportée ou acceptée.

Des méthodes ont certes déjà été proposées en vue de traiter l'adiposité, mais parmi celles-ci, seules en fait les méthodes reposant sur des traitements chirurgicaux, tels que la liposuccion, permettent actuellement d'obtenir des résultats véritablement satisfaisants. Toutefois, de tels traitements présentent bien évidemment comme inconvénient majeur de nécessiter la mise en oeuvre sur le corps humain d'opérations invasives par nature délicates, non sans risques et souvent coûteuses.

Par ailleurs, il existe des crèmes et des gels appropriés pour des applications topiques locales. Toutefois, les actifs amincissants apportés par cette voie sont immédiatement libérés dans la peau et ne présentent donc pas d'effet retard, ce qui minimise leur efficacité.

Il existe donc aujourd'hui dans l'état de l'art un fort besoin de disposer d'un procédé de traitement cosmétique "doux" et rapide, de type non chirurgical, permettant de lutter efficacement contre l'adiposité humaine ou contre la cellulite, en vue notamment d'obtenir un effet d'amincissement et/ou d'affinement de la peau ou de la silhouette, et ceci de telle sorte que les actifs amincissants diffusent lentement dans la peau.

La présente invention vise justement à la satisfaction d'un tel besoin.

La demanderesse a trouvé de manière surprenante qu'une composition comprenant un actif amincissant associé à un polymère filmogène permettait un traitement local particulièrement efficace de l'adiposité et de la cellulite, notamment par pulvérisation de la dite composition sur la zone à traiter.

La présente invention se rapporte donc à une composition amincissante sous forme de spray comprenant, dans un milieu physiologiquement acceptable contenant au moins un alcool en C₁-C₆, au moins un polymère filmogène hydrosoluble et au moins un actif amincissant.

La composition obtenue permet de former un film, c'est-à-dire une mince pellicule homogène, qui est invisible sur la peau et qui a la propriété d'épouser parfaitement le relief de la peau. Ce film est gorgé d'actifs amincissants qui vont diffuser lentement dans la peau afin de maximaliser leurs effets. Le film, après séchage, n'est pas du tout collant, tout en étant mou, souple, élastique et flexible sur la peau sur laquelle il est déposé. Il adhère bien à la peau tout en suivant ses mouvements sans se craqueler et/ou se décoller et sans provoquer de sensation de tiraillement ni de dessèchement.

Par ailleurs, le film formé sur la peau a la propriété de pouvoir être éliminé à l'eau, par exemple lors de la toilette. En outre, il peut être conservé plusieurs heures sans inconvénients.

Afin d'être pulvérisable, il est préférable que la composition de l'invention soit fluide et s'écoule rapidement sous son propre poids, à température ambiante (25°C). Avantageusement, elle présente une viscosité inférieure à 50 cPs, mesurée au viscosimètre à 25°C, avec une aiguille tournant dans la composition à une vitesse de 60 tr/min. Le viscosimètre est en particulier du type Brookfield LVTD équipé d'un mobile 2.

On entend dans la présente invention par « polymère filmogène » tout polymère susceptible de former un film continu et homogène sur la peau et utilisé en quantité suffisante pour former un tel film. Les polymères épaississants tels que par exemple les carbomers (comme les produits commercialisés sous la dénomination « Carbopol » par la société Goodrich) influent sur la rhéologie de la composition en l'épaississant, mais ne permettent pas de former un film continu sur la peau.

Dans la composition selon l'invention, on peut utiliser tout polymère filmogène hydrosoluble connu en soi. Par hydrosoluble, on entend un polymère soluble dans de l'eau et/ou des alcools inférieurs, c'est-à-dire les alcools comportant de un à six atomes de carbone, tels que l'éthanol. Le polymère filmogène hydrosoluble peut être choisi parmi les polymères anioniques, cationiques, amphotères, non ioniques et leurs mélanges, et se trouver éventuellement sous forme solubilisée ou sous forme de dispersions de particules solides de polymère.

Les polymères filmogènes cationiques utilisables selon la présente invention sont de préférence choisis parmi les polymères comportant des groupements amine primaire, secondaire, tertiaire et/ou quaternaire, ces groupements faisant partie de la chaîne polymère ou étant directement reliés à celle-ci, les dits polymères ayant un poids moléculaire allant d'environ 500 à environ 5.000.000 et de préférence d'environ 1000 à environ 3.000.000.

Parmi ces polymères cationiques, on peut citer plus particulièrement les polymères suivants :
(1) les homopolymères ou copolymères dérivés d'esters ou d'amides acryliques ou méthacryliques et comportant au moins un des motifs de formules suivantes : 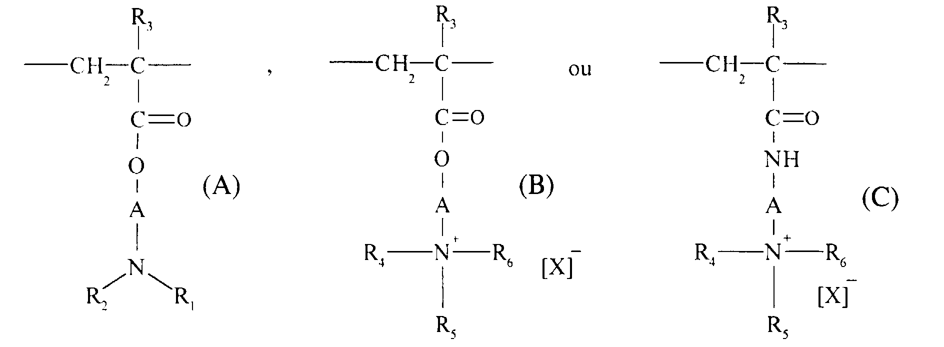 dans lesquelles :
   R₃ désigne un atome d'hydrogène ou un radical méthyle ;
   A est un groupe alkyle linéaire ou ramifié ayant de 1 à 6 atomes de carbone ou un groupe hydroxyalkyle ayant de 1 à 4 atomes de carbone ;
   R₄, R₅, R₆, identiques ou différents, représentent un groupe alkyle ayant de 1 à 18 atomes de carbone ou un radical benzyle ;
   R₁ et R₂ représentent un atome d'hydrogène ou un groupe alkyle ayant de 1 à 6 atomes de carbone ;
   X désigne un anion méthosulfate ou un halogénure tel que chlorure ou bromure.

   Les copolymères de la famille (1) contiennent en outre un ou plusieurs motifs dérivant de comonomères pouvant être choisis dans la famille des acrylamides, méthacrylamides, diacétones acrylamides, acrylamides et méthacrylamides substitués sur l'azote par des radicaux alkyle en C₁-C₈, des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylamino-éthylméthacrylate quaternisé au sulfate de diméthyle ou avec un halogénure de diméthyle, tels que celui vendu sous la dénomination HERCOFLOC par la société Hercules ;
   - les copolymères d'acrylamide et de chlorure de méthacryloyloxyéthyltriméthylammonium décrits par exemple dans le document EP-A-080976 et vendus sous la dénomination BINA QUAT P 100 par la société Ciba Geigy ;
   - le copolymère d'acrylamide et de méthosulfate de méthacryloyloxyéthyltriméthylammonium vendu sous la dénomination RETEN par la société Hercules ;
   - les copolymères vinylpyrrolidone / acrylate ou méthacrylate de dialkylaminoalkyle quaternisés ou non, tels que les produits vendus sous la dénomination GAFQUAT par la société ISP comme par exemple GAFQUAT 734 ou GAFQUAT 755, ou bien les produits dénommés COPOLYMER 845, 958 et 937. Ces polymères sont décrits en détail dans les documents FR-A-2,077,143 et FR-A-2,393,573 ;
   - les terpolymères méthacrylate de diméthylaminoéthyle/ vinylcaprolactame/ vinylpyrrolidone, tels que le produit vendu sous la dénomination GAFFIX VC 713 par la société ISP ;
   - et le copolymère vinylpyrrolidone / méthacrylamide de diméthylaminopropyle quaternisé, tel que le produit vendu sous la dénomination GAFQUAT HS 100 par la société ISP.
(2) les polysaccharides quaternisés tels que ceux décrits plus particulièrement dans les documents US-A-3,589,578 et US-A-4,031,307, tels que les gommes de guar contenant des groupements cationiques trialkylammonium. De tels produits sont commercialisés notamment sous les dénominations JAGUAR C13 S, JAGUAR C 15, JAGUAR C 17 par la société Meyhall ;
(3) les copolymères quaternaires de vinylpyrrolidone et de vinylimidazole ;
(4) les chitosanes ou leurs sels ; les sels utilisables sont en particulier les acétate, lactate, glutamate, gluconate ou pyrrolidone carboxylate de chitosane.
   Parmi ces composés, on peut citer le chitosane ayant un taux de désacétylation de 90,5% en poids, vendu sous la dénomination KYTAN BRUT STANDARD par la société Aber Technologies, le pyrrolidone carboxylate de chitosane vendu sous la dénomination KYTAMER PC par la société Amerchol ;
(5) les dérivés de cellulose cationiques tels que les copolymères de cellulose ou de dérivés de cellulose greffés avec un monomère hydrosoluble comportant un ammonium quaternaire et décrits notamment dans le document US-A-4,131,576, tels que les hydroxyalkylcelluloses comme les hydroxyméthyl-, hydroxyéthyl- ou hydroxypropyl-celluloses greffées notamment avec un sel de méthacryloyloxyéthyltriméthylammonium, de méthacrylamidopropyltriméthylammonium, de diméthyl-diallylammonium. Les produits commercialisés répondant à cette définition sont plus particulièrement les produits vendus sous la dénomination CELQUAT L 200 et CELQUAT H 100 par la Société National Starch.

Les polymères filmogènes anioniques utilisables dans la composition de l'invention sont des polymères comportant des groupements dérivés d'acides carboxylique, sulfonique ou phosphorique et ils ont un poids moléculaire allant environ de 500 à 5.000.000. On peut citer notamment comme polymères filmogènes anioniques ceux où les groupements carboxyliques sont apportés par des monomères mono- ou diacides carboxyliques insaturés tels que ceux répondant à la formule : 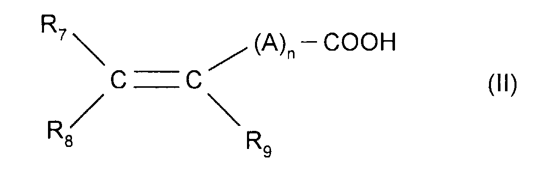 dans laquelle n est un nombre entier de 0 à 10, A₁ désigne un groupement méthylène, éventuellement relié à l'atome de carbone du groupement insaturé ou au groupement méthylène voisin lorsque n est supérieur à 1 par l'intermédiaire d'un hétéroatome tel que l'oxygène ou le soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule (II), un radical alkyle inférieur désigne de préférence un groupement ayant 1 à 4 atomes de carbone et, en particulier, méthyle et éthyle.

Les polymères filmogènes anioniques à groupements carboxyliques préférés selon l'invention sont :
A) Les homo- ou copolymères d'acide acrylique ou méthacrylique ou leurs sels et en particulier les produits vendus sous les dénominations VERSICOL E ou K par la société Allied Colloid, ceux vendus sous les dénominations ULTRAHOLD par la société BASF, les copolymères d'acide acrylique et d'acrylamide vendus sous la forme de leur sel de sodium sous les dénominations RETEN 421, 423 ou 425 par la Société Hercules, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et/ou éventuellement réticulés, tels que décrits en particulier dans les documents FR-A-1,222,944 et DE-A-2,330,956 ; les copolymères de ce type comportant dans leur chaîne un motif acrylamide éventuellement N-alkylé et/ou hydroxyalkylé, tels que décrits notamment dans les documents LU-A-75370 et LU-A-75371 ou ceux proposés sous la dénomination QUADRAMER par la Société American Cyanamid. On peut également citer les copolymères d'acide acrylique et de méthacrylate d'alkyle en C₁-C₄, les terpolymères de vinylpyrrolidone, d'acide acrylique et de méthacrylate d'alkyle en C₁-C₂₀, par exemple de lauryle, tel que celui vendu par la société ISP sous la dénomination ACRYLIDONE LM, et les terpolymères acide méthacrylique/ acrylate d'éthyle/ acrylate de tertiobutyle tels que le produit vendu sous la dénomination LUVIMER 100 P par la société BASF.
C) Les copolymères dérivés d'acide crotonique, tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée, tels que ceux comportant au moins 5 atomes de carbone, ces polymères pouvant éventuellement être greffés et réticulés, ou encore un ester vinylique, allylique ou méthallylique d'un acide carboxylique α- ou β-cyclique. De tels polymères sont décrits entre autres dans les documents FR-A-1,222,944, FR-A-1,580,545, FR-A-2,265,782, FR-A-2,265,781, FR-A-1,564,110 et FR-A-2,439,798. Des produits commerciaux entrant dans cette classe sont les résines 28-29-30, 26-13-14 et 28-13-10 vendues par la société National Starch.
D) Les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les documents US-A-2,047,398, US-A-2,723,248, US-A-2,102,113, GB-A-839 805, et notamment ceux vendus sous les dénominations GANTREZ AN ou ES par la société ISP ;
   - les copolymères comprenant (i) un ou plusieurs anhydrides maléique, citraconique, itaconique et (ii) au moins un monomère choisi parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées. Ces polymères sont par exemple décrits dans les documents FR-A-2,350,384 et FR-A-2,357,241.
E) Les polyacrylamides comportant des groupements carboxylates.

Les polymères comprenant les groupements sulfoniques sont des polymères comportant des motifs vinylsulfonique, styrènesulfonique, naphtalènesulfonique ou acrylamidoalkylsulfonique.

Ces polymères peuvent être notamment choisis parmi :
- les sels de l'acide polyvinylsulfonique ayant un poids moléculaire allant environ de 1.000 à 100.000, ainsi que les copolymères avec un comonomère insaturé tel que les acides acrylique ou méthacrylique et leurs esters ainsi que l'acrylamide ou ses dérivés, les éthers vinyliques et la vinylpyrrolidone ;
- les sels de l'acide polystyrène sulfonique, les sels de sodium ayant un poids moléculaire d'environ 500.000 et d'environ 100.000 vendus respectivement sous les dénominations Flexan 500 et Flexan 130 par la société National Starch. Ces composés sont décrits dans le document FR-A-2,198,719 ;
- les sels d'acides polyacrylamide sulfoniques tels que ceux mentionnés dans le document US-A-4,128,631, et plus particulièrement l'acide polyacrylamidoéthylpropanesulfonique vendu sous la dénomination COSMEDIA POLYMER HSP 1180 par la société Henkel.

Selon l'invention, les polymères filmogènes anioniques sont de préférence choisis parmi les copolymères d'acide acrylique, tels que le terpolymère acide acrylique / acrylate d'éthyle / N-tertiobutylacrylamide vendu sous la dénomination ULTRAHOLD STRONG par la société BASF ; les copolymères dérivés d'acide crotonique tels que les terpolymères acétate de vinyle / tertio-butyl benzoate de vinyle / acide crotonique et les terpolymères acide crotonique / acétate de vinyle / néododécanoate de vinyle vendus sous la dénomination Résine 28-29-30 par la société National Starch ; les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters, tels que le copolymère méthylvinyléther/anhydride maléique monoestérifié vendu sous la dénomination GANTREZ ES 425 par la société ISP ; les copolymères d'acide méthacrylique et de méthacrylate de méthyle vendus sous la dénomination EUDRAGIT L par la société Rohm Pharma ; le copolymère d'acide méthacrylique et d'acrylate d'éthyle vendu sous la dénomination LUVIMER MAEX ou MAE par la société BASF ; le copolymère acétate de vinyle / acide crotonique vendu sous la dénomination LUVISET CA 66 par la société BASF et le copolymère acétate de vinyle/acide crotonique greffé par du polyéthylèneglycol vendu sous la dénomination ARISTOFLEX A par la société BASF.

Les polymères filmogènes anioniques les plus particulièrement préférés sont choisis parmi le copolymère méthylvinyléther / anhydride maléique mono estérifié vendu sous la dénomination GANTREZ ES 425 par la société ISP ; le terpolymère acide acrylique / acrylate d'éthyle / N-tertiobutylacrylamide vendu sous la dénomination ULTRAHOLD STRONG par la société BASF ; les copolymères d'acide méthacrylique et de méthacrylate de méthyle vendus sous la dénomination EUDRAGIT L par la société Rohm Pharma ; les terpolymères acétate de vinyle / tertio-butyl benzoate de vinyle / acide crotonique ; les terpolymères acide crotonique / acétate de vinyle / néododécanoate de vinyle vendus sous la dénomination Résine 28-29-30 par la société National Starch ; le copolymère acétate de vinyle / acide crotonique vendu sous la dénomination LUVISET CA 66 par la société BASF ; le copolymère d'acide méthacrylique et d'acrylate d'éthyle vendu sous la dénomination LUVIMER MAEX OU MAE par la société BASF ; le terpolymère de vinylpyrrolidone / acide acrylique / méthacrylate de lauryle vendu sous la dénomination ACRYLIDONE LM par la société ISP.

Les polymères filmogènes amphotères utilisables dans la composition de l'invention peuvent être choisis parmi les polymères comportant des motifs B et C répartis statistiquement dans la chaîne polymère, où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes. B et C peuvent également désigner une chaîne polymère cationique comportant des groupements amine primaire, secondaire, tertiaire et/ou quaternaire, dans laquelle au moins l'un des groupements amine porte un groupement carboxylique ou sulfonique relié par l'intermédiaire d'un radical hydrocarboné, ou bien B et C font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères filmogènes amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que l'acide acrylique, l'acide méthacrylique, l'acide maléïque, l'acide alpha-chloracrylique, et d'un monomère basique dérivé d'un composé vinylique substitué contenant au moins un atome basique tel que les dialkylaminoalkyl-méthacrylates et -acrylates, les dialkylaminoalkyl-méthacrylamides et -acrylamides. De tels composés sont décrits dans le document US-A-3,836,537.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

   Les acrylamides ou méthacrylamides N-substitués plus particulièrement préférés selon l'invention sont les groupements dont les radicaux alkyle contiennent de 2 à 12 atomes de carbone, et ce sont plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
   Les comonomères basiques préférés sont des méthacrylates d'aminoéthyle, de butylaminoéthyle, de N,N'-diméthylaminoéthyle, de N-tertio-butylaminoéthyle.
   Parmi ces polymères, on utilise plus particulièrement les copolymères à base d'octylacrylamide, d'acrylates et de butylaminoethylmethacrylate (nom CTFA : Octylacrylamide / acrylates / butylaminoethylmethacrylate copolymer), tels que les produits vendus sous la dénomination AMPHOMER ou LOVOCRYL 47 par la société National Starch.
(3) Les polyaminoamides réticulés et alcoylés partiellement ou totalement, dérivant de polyaminoamides de formule générale :

   ⁅CO―R₁₀-CO―Z⁆ (III)

   dans laquelle R₁₀ représente un radical divalent dérivé d'un acide dicarboxylique saturé, d'un acide aliphatique mono- ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical :

      ―NH―[(CH₂)x-NH]p― (IV)

      où x=2 et p=2 ou 3, ou bien x=3 et p=2, ce radical dérivant de la diéthylène triamine, de la triéthylène tétraamine ou de la dipropylène triamine ;
   b) dans les proportions de 0 à 40 moles %, le radical (IV) ci-dessus, dans lequel x=2 et p=1 et qui dérive de l'éthylènediamine, ou le radical dérivant de la pipérazine : 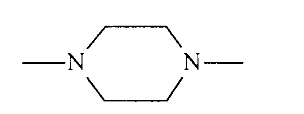
   c) dans les proportions de 0 à 20 moles % le radical -NH-(CH₂)₆-NH-dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

   Les acides carboxyliques saturés sont choisis de préférence parmi les acides ayant 6 à 10 atomes de carbone, tels que l'acide adipique, l'acide triméthyl-2,2,4-adipique et l'acide triméthyl-2,4,4-adipique, l'acide téréphtalique, les acides à double liaison éthylénique comme par exemple les acides acrylique, méthacrylique, itaconique.
   Les alcanes sultones utilisées dans l'alcoylation sont de préférence la propane sultone ou la butane sultone ; les sels des agents d'alcoylation sont de préférence les sels de sodium ou de potassium.
(4) Les polymères comportant des motifs zwittérioniques de formule : 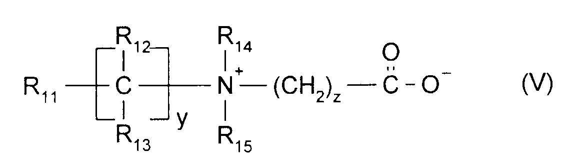 dans laquelle R₁₁ désigne un groupement insaturé polymérisable tel qu'un groupement acrylate, méthacrylate, acrylamide ou méthacrylamide, y et z représente un nombre entier de 1 à 3, R₁₂ et R₁₃ représentent un atome d'hydrogène, un radical méthyle, éthyle ou propyle, R₁₄ et R₁₅ représentent un atome d'hydrogène ou un radical alkyle, de telle façon que la somme des atomes de carbone dans R₁₄ et R₁₅ ne dépasse pas 10.

Les polymères comprenant de telles unités peuvent également comporter des motifs dérivés de monomères non zwittérioniques tels que l'acrylate ou le méthacrylate de diméthyl- ou diéthyl-aminoéthyle ou des alkyl-acrylates ou alkylméthacrylates, des acrylamides ou méthacrylamides ou l'acétate de vinyle.

A titre d'exemple, on peut citer le copolymère de méthacrylate de méthyle / diméthyl carboxyméthylammonioéthylméthacrylate de méthyle, tel que le produit vendu sous la dénomination DIAFORMER Z301 par la société Sandoz.
(5) Les polymères dérivés du chitosane comportant des motifs monomères répondant aux formules suivantes : 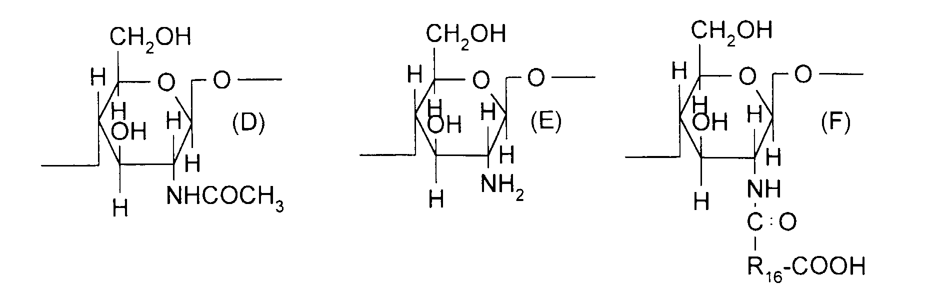 le motif D étant présent dans des proportions allant 0 à 30%, le motif E dans des proportions allant de 5 à 50 % et le motif F dans des proportions allant de 30 à 90 %, étant entendu que dans ce motif F, R₁₆ représente un radical de formule : 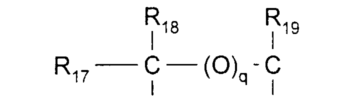 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine, éventuellement interrompus par un ou plusieurs atomes d'azote et/ou éventuellement substitués par un ou plusieurs groupes amine, hydroxyle, carboxyle, alcolylthio, sulfonique, un reste alcoylthio dont le groupe alcoyle porte un reste amino, l'un au moins des radicaux R₁₇, R₁₈ et R₁₉ étant dans ce cas un atome d'hydrogène ; ou si q=1, R₁₇, R₁₈ et R₁₉ représentent chacun un atome d'hydrogène, ainsi que les sels formés par ces composés avec des bases ou des acides.
(6) Les polymères dérivés de la N-carboxyalkylation du chitosane comme le N-carboxyméthylchitosane ou le N-carboxybutylchitosane vendu sous la dénomination EVALSAN par la société Jan Dekker.
(7) Les polymères répondant à la formule générale (VI), par exemple ceux décrits dans le document FR-A-1,400,366 : 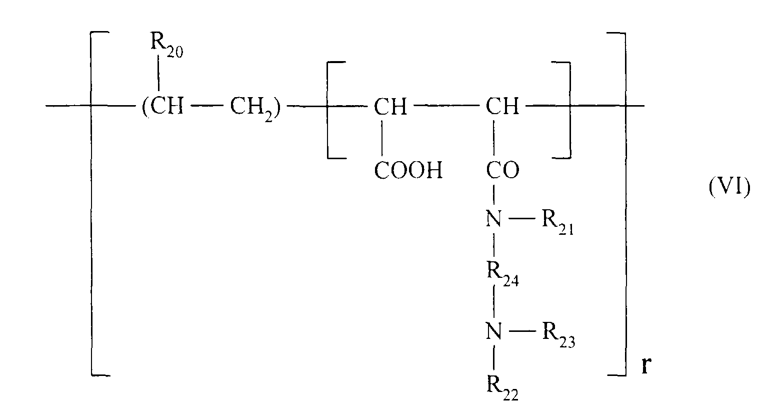 dans laquelle R₂₀ représente un atome d'hydrogène, un radical CH₃O, CH₃CH₂O, phényle, R₂₁ désigne l'hydrogène ou un radical alkyle inférieur tel que méthyle, éthyle, R₂₂ désigne l'hydrogène ou un radical alkyle inférieur tel que méthyle, éthyle, R₂₃ désigne un radical alkyle inférieur tel que méthyle, éthyle ou un radical répondant à la formule : -R₂₄-N(R₂₂)₂, R₂₄ représentant un groupement -CH₂-CH₂-, -CH₂-CH₂-CH₂-, -CH₂-CH(CH₃)-, R₂₂ ayant les significations mentionnées ci-dessus, ainsi que les homologues supérieurs de ces radicaux contenant jusqu'à 6 atomes de carbone.
(8) Les polymères amphotères du type -D-X-D-X- choisis parmi :
   a) les polymères obtenus par action de l'acide chloracétique ou le chloracétate de sodium sur les composés comportant au moins un motif de formule :

      -D-X-D-X-D- (VII)

      où D désigne un radical : 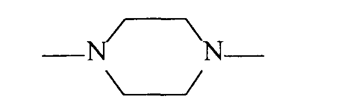 et X désigne le symbole E ou E', E ou E' identiques ou différents désignent un radical bivalent qui est un radical alkylène à chaîne droite ou ramifiée comportant jusqu'à 7 atomes de carbone dans la chaîne principale, non substituée ou substituée par des groupements hydroxyle et pouvant comporter en outre des atomes d'oxygène, d'azote, de soufre, 1 à 3 cycles aromatiques et/ou hétérocycliques, les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) Les polymères de formule :

      -D-X-D-X- (VII')

      où D désigne un radical : 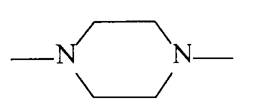 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)-vinyléther / anhydride maléique modifié partiellement par semi-amidification avec une N,N-dialkylaminoalkylamine telle que la N,N-diméthylaminopropylamine ou par semi-estérification avec une N,N-dialcanolamine. Ces copolymères peuvent également comporter d'autres comonomères vinyliques tels que le vinylcaprolactame.

Les polymères filmogènes amphotères particulièrement préférés selon l'invention sont ceux de la famille (3), tels que les copolymères ayant la dénomination CTFA "Octylacrylamide/acrylates/butylaminoethylmethacrylate copolymer", comme les produits vendus sous les dénominations AMPHOMER, AMPHOMER LV 71 ou LOVOCRYL 47 par la société National Starch, et ceux de la famille (4) tels que le copolymère de méthacrylate de méthyle / diméthylcarboxyméthylammonioéthylméthacrylate de méthyle, par exemple vendu sous la dénomination DIAFORMER Z301 par la société Sandoz.

Les polymères filmogènes non ioniques utilisables selon la présente invention sont choisis par exemple parmi :
- les homopolymères de vinylpyrrolidone tels que le produit commercialisé sous le nom LUVISKOL K30 par la société BASF;
- les copolymères de vinylpyrrolidone et d'acétate de vinyle, tels que le produit commercialisé sous le nom LUVISKOL VA 64 par la société BASF ;
- les polyalkyloxazolines telles que les polyéthyloxazolines proposées par la société Dow Chemical sous les dénominations PEOX 50 000, PEOX 200 000 et PEOX 500 000 ;
- les homopolymères d'acétate de vinyle, tels que le produit proposé sous le nom de APPRETAN EM par la société Hoechst ou celui proposé sous le nom de RHODOPAS A 012 par la société Rhone Poulenc ;
- les copolymères d'acétate de vinyle et d'ester acrylique tels que le produit proposé sous le nom de RHODOPAS AD 310 de Rhone Poulenc ;
- les copolymères d'acétate de vinyle et d'éthylène tels que le produit proposé sous le nom de APPRETAN TV par la société Hoechst ;
- les copolymères d'acétate de vinyle et d'ester maléïque par exemple de maléate de dibutyle tels que le produit proposé sous le nom de APPRETAN MB EXTRA par la société Hoechst ;
- les copolymères de polyéthylène et d'anhydride maléïque ;
- les homopolymères d'acrylates d'alkyle et les homopolymères de méthacrylates d'alkyle tels que le produit proposé sous la dénomination MICROPEARL RQ 750 par la société Matsumoto ou le produit proposé sous la dénomination LUHYDRAN A 848 S par la société BASF ;
- les copolymères d'esters acryliques tels que par exemple les copolymères d'acrylates d'alkyle et de méthacrylates d'alkyles tels que les produits proposés par la société Rohm & Haas sous les dénominations PRIMAL AC-261 K et EUDRAGIT NE 30 D, par la société BASF sous les dénominations ACRONAL 601, LUHYDRAN LR 8833 ou 8845, par la société Hoechst sous les dénominations APPRETAN N 9213 ou N9212 ;
- les copolymères d'acrylonitrile et d'un monomère non ionique choisi par exemple parmi le butadiène et les (méth)acrylates d'alkyle ; on peut citer les produits proposés sous les dénominations NIPOL LX 531 B par la société Nippon Zeon ou ceux proposés sous la dénomination CJ 0601 B par la société Rohm & Haas ;
- les polyuréthannes tels que les produits proposés sous les dénominations ACRYSOL RM 1020 ou ACRYSOL RM 2020 par la société Rohm & Haas, les produits URAFLEX XP 401 UZ, URAFLEX XP 402 UZ par la société DSM Resins ;
- les copolymères d'acrylate d'alkyle et d'uréthanne tels que le produit 8538-33 par la société National Starch ;
- les polyamides tels que le produit ESTAPOR LO 11 vendu par la société Rhône Poulenc ;
- les gommes de guar non ioniques chimiquement modifiées ou non modifiées. Comme gommes de guar non ioniques non modifiées, on peut citer par exemple les produits vendus sous la dénomination VIDOGUM GH 175 par la société Unipectine et sous la dénomination JAGUAR C par la société Meyhall. Les gommes de guar non-ioniques modifiées utilisables selon l'invention sont de préférence modifiées par des groupements hydroxyalkyle en C₁-C₆. On peut mentionner à titre d'exemple, les groupements hydroxyméthyle, hydroxyéthyle, hydroxypropyle et hydroxybutyle.

Ces gommes de guar sont bien connues et peuvent par exemple être préparées en faisant réagir des oxydes d'alcènes correspondants, tels que par exemple des oxydes de propylène, avec la gomme de guar de façon à obtenir une gomme de guar modifiée par des groupements hydroxypropyle. De telles gommes de guar non-ioniques éventuellement modifiées par des groupements hydroxyalkyle sont par exemple vendues sous les dénominations JAGUAR HP8, JAGUAR HP60 et JAGUAR HP120, JAGUAR DC 293 et JAGUAR HP 105 par la société Meyhall, ou sous la dénomination GALACTASOL 4H4FD2 par la société Aqualon.

Les radicaux alkyle des polymères non ioniques décrits ci-dessus ont de 1 à 6 atomes de carbone sauf mention contraire.

Selon l'invention, on peut également utiliser les polymères filmogènes de type siliconés greffés comprenant une portion polysiloxane et une portion constituée d'une chaîne organique non-siliconée, l'une des deux portions constituant la chaîne principale du polymère et l'autre étant greffée sur la dite chaîne principale. Ces polymères sont par exemple décrits dans les documents EP-A-0 412 704, EP-A-0 412 707, EP-A-0 640 105, WO-A-95/00578, EP-A-0 582 152, WO-A-93/23009, US-A-4,693,935, US-A-4,728,571 et US-A-4,972,037. Ces polymères sont de préférence anioniques ou non ioniques.

De tels polymères sont par exemple les copolymères susceptibles d'être obtenus par polymérisation radicalaire à partir du mélange de monomères constitué par :
a) 50 à 90 % en poids d'acrylate de tertiobutyle ;
b) 0 à 40 % en poids d'acide acrylique ;
c) 5 à 40 % en poids de macromère siliconé de formule : 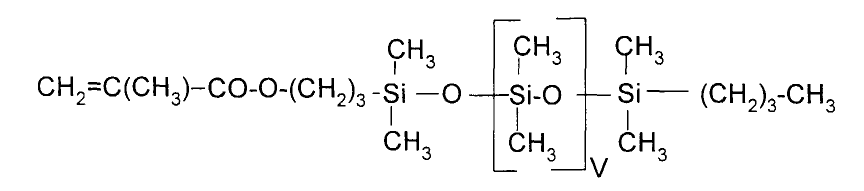 dans laquelle v est un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle, et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon un mode préféré de réalisation de la présente invention, les polymères filmogènes sont de préférence des polymères anioniques, amphotères ou non ioniques.

Les polymères filmogènes anioniques ou amphotères peuvent être si nécessaire neutralisés partiellement ou totalement. Les agents de neutralisation sont par exemple la soude, la potasse, l'amino-2 méthyl-2 propanol-1 (ou aminométhyl propanol), la monoéthanolamine, la triéthanolamine ou la triisopropanolamine, les acides minéraux ou organiques tels que l'acide chlorhydrique ou l'acide citrique.

Le ou les polymères filmogènes sont présents dans la composition de l'invention en une concentration telle que la composition obtenue forme un film sur la peau. Cette concentration varie selon le ou les polymères utilisés et peut aller par exemple de 0,05 à 20 % en poids de matière active, de préférence de 0,1 à 10 % et mieux de 1 à 10 % en poids de matière active par rapport au poids total de la composition.

Le milieu physiologiquement acceptable est un milieu compatible avec la peau. Il comprend au moins un monoalcool en C₁-C₆, et peut être constitué par un monoalcool en C₁-C₆, de l'eau et/ou un solvant physiologiquement acceptable. Ce solvant peut être choisi notamment parmi les polyalcools, les éthers de glycol ou les esters d'acides gras.

Parmi les monoalcools en C₁-C₆, on peut citer l'éthanol et l'isopropanol. Comme solvants, on peut citer les polyalcools tels que le diéthylèneglycol, les éthers de glycol tels que les monoalkyléthers de glycol, de diéthylèneglycol, de propylèneglycol ou de dipropylèneglycol. L'éthanol est particulièrement préféré comme monoalcool. Le monoalcool et plus particulièrement l'éthanol peut être présent en une quantité allant par exemple de 50 à 99 % en poids, de préférence de 70 à 98 % en poids et mieux de 76 à 98 % en poids par rapport au poids total de la composition.

Selon un mode particulier de réalisation de l'invention, la composition amincissante comprend un mélange d'éthanol et d'eau, celle-ci étant présente en une quantité allant de 5 à 25 % en poids par rapport au poids total de la composition et l'éthanol étant présent en une quantité complémentaire pour avoir une composition complétée à 100 % en poids compte tenu des autres composés présents, et par exemple en une quantité allant de 70 à 98 % en poids par rapport au poids total de la composition.

La composition de l'invention peut également contenir au moins un additif choisi parmi les épaississants, les plastifiants du polymère filmogène, les émollients, les hydratants tels que la glycérine, les esters d'acides gras, les esters d'acides gras et de glycérol, les silicones volatiles ou non volatiles, solubles ou insolubles dans la composition, les parfums, les conservateurs, les filtres solaires, les protéines, les vitamines, les polymères, les huiles végétales, animales, minérales ou synthétiques et tout autre additif classiquement utilisé dans les compositions cosmétiques.

Comme plastifiants, on peut citer par exemple les citrates d'alkyle et notamment le citrate de triéthyle, et les alkyléthers de glycol tels que le mono-méthyl-éther de tripropylène glycol.

Ces additifs sont présents dans la composition selon l'invention dans des proportions pouvant aller de 0 à 20 % en poids par rapport au poids total de la composition. La quantité précise de chaque additif est fonction de sa nature et est déterminée facilement par l'homme de l'art. Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Selon un mode préféré de réalisation de l'invention, la composition selon l'invention est exempte de tensioactif, notamment pour des raisons d'innocuité, la présence de tensioactif dans le film formé sur la peau pouvant entraîner une irritation de la peau.

Comme actifs amincissants, on peut utiliser dans la composition de l'invention tous les composés connus en soi comme présentant une activité plus ou moins marquée dans le domaine de la lutte contre l'adiposité et/ou la cellulite. Ils peuvent être choisis par exemple parmi :
1) les inhibiteurs de phosphodiestérase, tels que :
   - les dérivés xanthiques comme la caféine et ses dérivés, notamment les 1-hydroxyalkylxanthines décrites dans le document FR-A-2,617,401, la caféine citrate, la théophylline et ses dérivés, la théobromine, l'acéfylline, l'aminophylline, le chloroéthylthéophylline, le diprofylline, le diniprophylline, l'étamiphylline et ses dérivés, l'étofylline, la proxyphylline ;
   - les associations contenant des dérivés xanthiques, comme l'association de caféine et de silanol (dérivé méthylsilanetriol de caféine), et par exemple le produit commercialisé par la société Exsymol sous la dénomination caféisilane C ;
   - les composés d'origine naturelle contenant des bases xanthiques et notamment de la caféine, tels que les extraits de thé, de café, de guarana, de maté, de cola *(Cola Nitida)* et notamment l'extrait sec de fruit de guarana *(Paulina sorbilis)* contenant 8 à 10 % de caféine ;
   - l'éphédrine et ses dérivés qui peuvent notamment se retrouver à l'état naturel dans les plantes telles que le Ma Huang (Ephedra plant)
2) les extraits végétaux et les extraits d'origine marine, qui sont soit actifs sur les récepteurs à inhiber, tels que les α2-bloqueurs, les NPY-bloqueurs, soit actifs pour stimuler les récepteurs β et les protéines G, conduisant à l'activation de l'adénylcyclase, soit encore ceux agissant sur la lipoprotéine lipase, inhibant la captation du glucose ou agissant sur d'autres enzymes favorables à la lipolyse et/ou défavorables à la lipogénèse. Comme extraits végétaux de ce type, on peut citer par exemple :
   - le *Garcinia Cambogia,*
   - les extraits de *Bupleurum chinensis,*
   - les extraits de lierre grimpant *(Hedera Helix),* d'arnica *(Arnica Montana L),* de romarin *(Rosmarinus officinalis N),* de souci *(Calendula officinalis),* de sauge *(Salvia officinalis L),* de ginseng *(Panax ginseng),* de millepertuis *(Byperycum Perforatum),* de fragon *(Ruscus aculeatus L),* d'ulmaire *(Filipendula ulmaria L),* d'orthosiphon *(Orthosiphon Stamincus Benth),* de bouleau *(Betula alba),*
   - les extraits de ginkgo biloba,
   - les extraits de prêle,
   - les extraits d'escine,
   - les extraits de cangzhu,
   - les extraits de *chrysanthellum indicum,*
   - les extraits des plantes du genre *Armeniacea, Atractylodis Platicodon, Sinommenum, Pharbitidis, Flemingia,*
   - les extraits de *Coleus* tels que *C. Forskohlii, C. blumei, C. esquirolii, C. scutellaroides, C. xanthantus* et *C. Barbatus,* tel que l'extrait de racine de *Coleus Barbatus* contenant 60 % de forskoline,
   - les extraits de Ballote,
   - les extraits de *Guioa,* de *Davallia,* de *Terminalia,* de *Barringtonia,* de *Trema, d'Antirobia.*
Comme extrait d'origine marine on peut citer :
   - les extraits d'algues, tels que le rhodystérol ou l'extrait de *Laminaria Digitata* commercialisé sous la dénomination PHYCOX75 par la société Secma,
   - les protamines et leurs dérivés tels que ceux décrits dans le document FR-A-2,758,724.
3) Les actifs amincissants qui se lient aux PPARs (Peroxisome Proliferator activated receptor), tels que ceux décrits dans les documents WO-A-97/10813 et WO-96/33724, ou les actifs qui bloquent la différenciation des cellules précurseurs d'adipocytes dans le tissu adipeux.

La quantité d'actif(s) amincissant(s) peut varier dans une large mesure et dépend de la nature de ou des actifs utilisés. De manière générale, le ou les actifs amincissants sont présents en une concentration allant de 0,05 à 20 % et de préférence de 0,1 à 10 % en poids par rapport au poids total de la composition.

On peut ajouter à ces actifs amincissants des actifs qui vont compléter l'action des actifs amincissants, et notamment :
1) les actifs agissant sur la microcirculation (vasoprotecteur ou vasodilatateur), tels que les flavonoïdes, les ruscogénines, les esculosides, l'escine extraite du marron d'Inde, les nicotinates, l'héperidine méthyl chalcone, le petit houx, les huiles essentielles de lavande ou de romarin ;
2) les actifs raffermissants et/ou antiglycant (empêchant la fixation du sucre sur les fibres de collagène), tels que les extraits de *centella asiatica* et de *siegesbeckia,* qui stimulent la synthèse de collagène, le silicium, l'amadorine (anti-glycation), la vitamine C et ses dérivés, et le rétinol et ses dérivés.

La quantité de ces actifs peut varier dans une large mesure. De manière générale, ces actifs sont présents en une concentration allant de 0,01 à 15 % et de préférence de 0,05 à 10 % en poids par rapport au poids total de la composition.

La composition selon l'invention est particulièrement appropriée pour une utilisation sous forme de spray (forme vaporisée). Après pulvérisation, le solvant s'évapore laissant sur la peau le polymère filmogène qui va permettre la diffusion de actifs amincissants. Pour assurer une application sous forme de spray, la composition peut être conditionnée dans un flacon pompe ou dans un récipient aérosol avec un gaz propulseur approprié qui peut être choisi parmi les hydrocarbures volatils tels que le n-butane, le propane, l'isobutane, le pentane, un hydrocarbure chloré et/ou fluoré, et leurs mélanges. On peut également utiliser en tant qu'agent propulseur le gaz carbonique, le protoxyde d'azote, le diméthyléther, l'azote, l'air comprimé et leurs mélanges.

La composition, objet de la présente invention trouve ainsi des applications particulièrement utiles et intéressantes dans le domaine des traitements cosmétiques visant à obtenir des effets locaux d'amincissement et/ou d'affinement de la peau ou de la silhouette (notamment hanches, fesses, cuisses, ventre et visage), et notamment pour lutter contre l'adiposité et/ou la cellulite et/ou la peau d'orange et/ou pour obtenir un effet d'amincissement du corps et/ou du visage.

Aussi, l'invention a encore pour objet l'utilisation cosmétique de la composition telle que définie ci-dessus, pour lutter contre l'adiposité, la cellulite et/ou la peau d'orange, et/ou obtenir un effet d'amincissement du corps et/ou du visage.

L'un des grands avantages de la présente invention réside dans la possibilité de pouvoir procéder, chaque fois que nécessaire ou souhaitable, à des traitements "doux" très localisés et sélectifs grâce au mode d'application locale par pulvérisation.

Pour obtenir des effets notables, les fréquences d'administration ou d'application des compositions selon l'invention, qui peuvent être bien entendu variables selon les quantités d'actifs mises en oeuvre à chaque opération, sont de l'ordre de une à deux fois par jour. Le traitement est ensuite poursuivi régulièrement, pendant plusieurs jours, de préférence pendant plusieurs semaines, voire plusieurs mois. Il n'y a en fait aucun inconvénient ou contre-indication à pratiquer continuellement et quotidiennement sur le corps le traitement selon l'invention.

Les exemples ci-après de compositions selon l'invention sont donnés à titre d'illustration et sans caractère limitatif. Les quantités y sont données en % en poids, sauf mention contraire.

### Exemple 1 : Spray amincissant

- Copolymère d'acétate de vinyle/acide crotonique (90/10) (LUVISET CA 66 de BASF) 4,5 %
- Aminométhylpropanol 0,48 %
- Extrait de *Cola Nitida* 0,25 %
- Extrait sec de fruit de guarana 0,1 %
- Extrait de racine de *Coleus Barbatus* contenant 60 % de forskoline 0,1 %
- Ethanol 94,57 %

Mode opératoire : on saupoudre le copolymère dans l'alcool contenant l'aminométhylpropanol tout en agitant, puis on additionne les extraits végétaux l'un après l'autre.

Le produit obtenu peut être appliqué sous forme de spray sur les zones cellulitiques.

### Exemple 2 : Spray amincissant

- Copolymère d'acétate de vinyle/acide crotonique (90/10) (LUVISET CA 66 de BASF) 4,5 %
- Caféine 2 %
- Extrait de fragon *(Ruscus)* 0,5 %
- Aminométhylpropanol 0,48 %
- Extrait de Ginkgo biloba 0,2 %
- Ethanol 76 %
- Eau 16,32 %

Mode opératoire : on saupoudre le copolymère dans l'alcool contenant l'aminométhylpropanol tout en agitant, puis on ajoute l'eau et, après dissolution du copolymère, on ajoute la caféine. On additionne ensuite les extraits végétaux l'un après l'autre.

Le produit obtenu peut être appliqué sous forme de spray sur les zones cellulitiques.

### Exemple 3 : Spray amincissant

- Copolymère d'acétate de vinyle/acide crotonique (90/10) (LUVISET CA 66 de BASF) 4,5 %
- Caféine 2 %
- Ethanol 76 %
- Eau 17,02 %

Mode opératoire : on saupoudre le copolymère dans l'alcool tout en agitant, puis on ajoute l'eau et, après dissolution du copolymère, on ajoute la caféine.

Le produit obtenu peut être appliqué sous forme de spray sur les zones cellulitiques.

### Exemple 4 : Spray amincissant

- Polymère de vinylpyrrolidone (LUVISKOL K30 de BASF) 3,5 %
- Caféine 2 %
- Extrait de racine de *Coleus Barbatus* contenant 60 % de forskoline 0,1 %
- Escine 0,2 %
- Eau 20 %
- Citrate de triéthyle 0,2 %
- Glycérine 1 %
- Ethanol qsp 100 %

Mode opératoire : on saupoudre le polymère dans l'alcool tout en agitant, puis on ajoute l'eau et, après dissolution du polymère, on ajoute la caféine. On additionne ensuite l'un après l'autre l'extrait de *Coleus Barbatus,* l'escine, le citrate de triéthyle et la glycérine.

Le produit obtenu peut être appliqué sous forme de spray sur les zones cellulitiques.

## Revendications

1. Composition amincissante sous forme de spray, comprenant, dans un milieu physiologiquement acceptable contenant au moins un monoalcool en C₁-C₆, au moins un polymère filmogène hydrosoluble et au moins un actif amincissant.

2. Composition selon la revendication 1, caractérisée en ce que le ou les polymères sont présents en une concentration allant de 1 à 10 % en poids de matière active, par rapport au poids total de la composition.

3. Composition selon la revendication 1 ou 2, caractérisée en ce que le polymère est choisi parmi les polymères anioniques, cationiques, amphotères, non ioniques et leurs mélanges.

4. Composition selon l'une quelconque des revendications précédentes, caractérisée en ce que le polymère est un polymère anionique choisi parmi :
- les polymères comportant des motifs carboxyliques dérivant de monomères mono- ou diacides carboxyliques insaturés de formule : 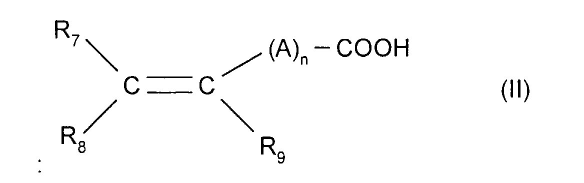 dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle ;
- les polymères comprenant des motifs dérivant d'acide sulfonique.

5. Composition selon l'une quelconque des revendications précédentes, caractérisée en ce que le polymère est un polymère anionique choisi parmi :
A) les homo- ou copolymères d'acide acrylique ou méthacrylique ou leurs sels, les copolymères d'acide acrylique et d'acrylamide et leurs sels, les sels de sodium d'acides polyhydroxycarboxyliques ;
B) les copolymères des acides acrylique ou méthacrylique avec un monomère monoéthylénique, éventuellement greffés sur un polyalkylène glycol et/ou éventuellement réticulés ; les copolymères de ce type comportant dans leur chaîne un motif acrylamide éventuellement N-alkylé et/ou hydroxyalkylé ; les copolymères d'acide acrylique et de méthacrylate d'alkyle en C₁-C₄ ;
C) les copolymères dérivés d'acide crotonique éventuellement greffés et/ou réticulés ;
D) les copolymères dérivés d'acides ou d'anhydrides carboxyliques monoinsaturés en C₄-C₈ choisis parmi :
- les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées ;
- les copolymères comprenant (i) un ou plusieurs anhydrides maléique, citraconique, itaconique et (ii) au moins un monomère choisi parmi les esters allyliques ou méthallyliques comportant éventuellement un ou plusieurs groupement acrylamide, méthacrylamide, α-oléfine, esters acryliques ou méthacryliques, acides acrylique ou méthacrylique ou vinylpyrrolidone dans leur chaîne,
les fonctions anhydrides de ces copolymères étant éventuellement monoestérifiées ou monoamidifiées.
E) les polyacrylamides comportant des groupements carboxylates.

6. Composition selon l'une quelconque des revendications précédentes, caractérisée en ce que le polymère est un polymère amphotère choisi parmi : les polymères comportant des motifs dérivant :
a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

7. Composition selon l'une quelconque des revendications précédentes, caractérisée en ce que le polymère est un polymère amphotère choisi parmi les copolymères d'octylacrylamide/ acrylates/ butylaminoethylmethacrylate et les copolymères de méthacrylate de méthyle / diméthyl carboxyméthylammonioéthyl-méthacrylate de méthyle.

8. Composition selon l'une quelconque des revendications précédentes, caractérisée en ce que le polymère est un polymère non ionique choisi parmi :
- les homopolymères de vinylpyrrolidone ;
- les copolymères de vinylpyrrolidone et d'acétate de vinyle ;
- les polyalkyloxazoline ;
- les homopolymères d'acétate de vinyle ;
- les copolymères d'acétate de vinyle et d'ester acrylique ;
- les copolymères d'acétate de vinyle et d'éthylène ;
- les copolymères d'acétate de vinyle et d'ester maléïque ;
- les copolymères de polyéthylène et d'anhydride maléfique ;
- les homopolymères d'acrylates d'alkyle et les homopolymères de méthacrylates d'alkyle ;
- les copolymères d'esters acryliques ;
- les copolymères d'acrylonitrile et d'un monomère non ionique ;
- les copolymères d'acrylate d'alkyle et d'uréthanne ;
- les polyamides ;
- les gommes de guar non ioniques chimiquement modifiées ou non modifiées.

9. Composition selon l'une quelconque des revendications précédentes, caractérisée en ce que le polymère est un polymère cationique choisi parmi :
- le copolymère d'acrylamide et de diméthylaminoéthyl méthacrylate quaternisé au sulfate de diméthyle ou avec un halogénure de diméthyle,
- les copolymères d'acrylamide et de chlorure de méthacryloyloxyéthyltriméthylammonium,
- le copolymère d'acrylamide et de méthosulfate de méthacryloyloxyéthyltriméthylammonium,
- les copolymères vinylpyrrolidone / acrylate ou méthacrylate de dialkylaminoalkyle quaternisés ou non,
- les terpolymères méthacrylate de diméthylaminoéthyle / vinylcaprolactame / vinylpyrrolidone,
- le copolymère vinylpyrrolidone / méthacrylamide de diméthylaminopropyle quaternisé,
- les polysaccharides quaternisés,
- les copolymères quaternaires de vinylpyrrolidone et de vinylimidazole,
- les chitosanes ou leurs sels,
- les dérivés de cellulose cationiques.

10. Composition selon l'une quelconque des revendications précédentes, caractérisée en ce qu'elle est exempte de tensioactif.

11. Composition selon l'une quelconque des revendications précédentes, caractérisée en ce que le monoalcool en C₁-C₆ est l'éthanol.

12. Composition selon l'une quelconque des revendications précédentes, caractérisée en ce qu'elle comprend de 70 à 98 % en poids de monoalcool en C₁-C₆ par rapport au poids total de la composition.

13. Composition selon l'une quelconque des revendications précédentes, caractérisée en ce que le milieu physiologiquement acceptable est un mélange d'éthanol et d'eau, la quantité d'eau allant de 5 à 25 % en poids par rapport au poids total de la composition.

14. Composition selon l'une quelconque des revendications précédentes, caractérisée en ce que l'actif amincissant est choisi parmi les inhibiteurs de phosphodiestérase, les extraits végétaux, les extraits d'origine marine, les actifs qui se lient aux PPARs, les actifs qui bloquent la différenciation des cellules précurseurs d'adipocytes.

15. Composition selon l'une quelconque des revendications précédentes, caractérisée en ce que l'actif amincissant est choisi parmi les dérivés xanthiques, les associations contenant des dérivés xanthiques, les composés d'origine naturelle contenant des dérivés xanthiques, l'éphédrine et ses dérivés, le *Garcinia Cambogia,* les extraits de *Bupleurum chinensis,* de lierre grimpant, d'arnica, de romarin, de souci, de sauge, de ginseng, de millepertuis, de fragon, d'ulmaire, d'orthosiphon, de bouleau, de ginkgo biloba, de prêle, d'escine, de cangzhu, de *chrysanthellum indicum,* de plantes, de *Coleus,* de Ballote, de *Guioa,* de *Davallia,* de *Terminalia,* de *Barringtonia,* de *Trema,* d'*Antirobia,* les extraits d'algues, les protamines et leurs dérivés.

16. Composition selon l'une quelconque des revendications précédentes, caractérisée en ce que la quantité d'actif(s) amincissant(s) va de 0,05 à 20 % en poids par rapport au poids total de la composition.

17. Composition selon l'une quelconque des revendications précédentes, caractérisée en ce qu'elle comprend au moins un actif choisi parmi les actifs, agissant sur la microcirculation, les actifs raffermissants et les actifs antiglycant.

18. Utilisation cosmétique de la composition selon l'une quelconque des revendications précédentes, pour lutter contre l'adiposité, la cellulite et/ou la peau d'orange, et/ou obtenir un effet d'amincissement du corps et/ou du visage.
